# EUROPÄISCHE PATENTANMELDUNG

(11) **EP 2 823 777 A1**
(43) Veröffentlichungstag der Anmeldung: **14.01.2015**
(21) Anmeldenummer: 14002325.0
(22) Anmeldetag: 08.07.2014
(51) Int. Cl.: A61B 17/14, A61B 17/00

(54) **Chirurgisches Bearbeitungswerkzeug für die Knochenchirurgie**

(30) Priorität: 12.07.2013 DE 102013011638
(71) Anmelder: Marks, Peter, 65468 Trebur (DE); Rahman, Sanny, 64572 Büttelborn (DE)
(72) Erfinder: Marks, Peter, 65468 Trebur (DE); Rahman, Sanny, 64572 Büttelborn (DE)
(74) Vertreter: Behrens, Helmut

(57) **Zusammenfassung**

Die Erfindung betrifft ein chirurgisches Bearbeitungswerkzeug (1) für die Knochenchirurgie, dass ein Halteelement (2) zur Verbindung mit einem Antrieb und mindestens einem Bearbeitungselement (7) zur spanabhebenden Bearbeitung von Knochen enthält, wobei mindestens das Bearbeitungselement (7) aus einem harten Werkstoff besteht. Die Erfindung ist dadurch gekennzeichnet, dass mindestens das Bearbeitungselement (7) aus einem biologisch abbaubaren Werkstoff besteht, der Milchsäure enthält.

## Beschreibung

Die Erfindung betrifft ein chirurgisches Bearbeitungswerkzeug für die Knochenchirurgie nach dem Oberbegriff des Patentanspruchs 1.

In der Knochenchirurgie werden häufig Bearbeitungswerkzeuge benutzt, durch die Knochenteile durch einen Sägevorgang voneinander getrennt werden, um z.B. künstliche Gelenke daran zu befestigen. Zur Bearbeitung von Knochen sind aber auch chirurgische Bohrer bekannt, um bei Knochenbrüchen Bohrlöcher für Befestigungsplatten an den Knochen anzubringen. Es sind als Bearbeitungswerkzeuge aber auch Fräß- oder Raspelvorrichtungen bekannt, um die vorhandenen Knochenoberflächenformen zu verändern. Dazu weisen die Bearbeitungswerkzeuge schneidenförmige Bearbeitungselemente auf, deren Werkstoff härter ist als der der zu bearbeitenden Knochenteile.

Ein derartiges chirurgisches Bearbeitungswerkzeug in Form einer Knochenraspel ist aus der EP 0 378 002 B1 bekannt. Dabei besteht die Raspel aus einem länglichen plattenförmigen Körper, der an einem Ende eine Befestigungseinrichtung aufweist, die als Achteckaussparung ausgebildet und die in einer Antriebsvorrichtung einsetzbar ist. An dem gegenüberliegenden Bereich des plattenförmigen Körpers sind eine Vielzahl von Raspelelementen angebracht, die jeweils aus sternförmig angeordneten Schlitzen bestehen, an denen nach außen abgebogene Schneiden angebracht sind. Um mit den Raspelelementen bei einem Knochen eine glatte Oberfläche zu erhalten, ist der plattenförmige Körper aus einem durchgehärteten rostfreien Stahl gefertigt. Dabei wird jedes Raspelelement durch Laserschneiden hergestellt, wobei danach die Schneidelemente nach außen gebogen werden, wodurch ein günstiger Schneidwinkel entsteht. Allerdings kann es beim Raspelbetrieb am zu bearbeitenden Knochen vorkommen, dass die scharfen Schneidkanten abbrechen oder abgerieben werden, so dass Metallteile in den menschlichen Körper gelangen, die im Grunde nicht mehr entfernbar sind und die die Verbindung zwischen dem Knochen und einer daran befestigten Protese ungünstig beeinträchtigen können.

Ein weiteres chirurgisches Bearbeitungswerkzeug im Form eines Bohrers zur Schädelperforation ist aus der DE 36 24 860 A1 bekannt. Dieser Bohrer besteht aus einem inneren und einem äußeren Bohrerteil, einer vorderen Bohrerkopfanordnung und einer hinteren Stütz- und Antriebsanordnung. Zum Durchbohren des Schädelknochens dient hauptsächlich der innere Bohrerteil, der mindestens drei Rippen oder Klingen enthält, die 120° versetzt sind und geneigte Schnittflächen aufweisen. Dabei besteht mindestens der innere Bohrerteil offensichtlich aus einer harten Eisenlegierung, um die Schädeldecke mittels der Schneiden aufbohren zu können. Da zum Aufbohren der innere Bohrer stark auf dem harten Schädelknochen gedrückt werden muss und mit Drehzahlen von 800 bis 1000 Umdrehungen pro Minute angetrieben wird, können dadurch Abrieb- und Metallpartikel von der Schneide ins Schädelinnere gelangen und sind dort nicht mehr entfernbar.

Als chirurgisches Bearbeitungswerkzeug ist aus der DE 101 00 630 C1 ein Sägeblatt für eine chirurgische Oszillationssäge bekannt. Dabei besteht das Sägeblatt aus einem flachen, langgestreckten Blechkörper aus Stahl, der an dem einen Ende eine Befestigungsaussparung und am anderen Ende eine gekrümmte Sägezahnreihe aufweist. Die Befestigungsaussparung wird mit einem Oszillationsantrieb verbunden, durch den das Sägeblatt um eine Oszillationsachse periodisch verdreht wird. Dadurch bewegt sich die gekrümmte Sägezahnreihe oszillierend um die angetriebene Oszillationsachse, wodurch die vorgesehenen Knochen zersägt werden. Damit die Knochenspäne aus dem Körper herausgeführt werden, besitzt das Sägeblatt acht konvex zur Oszillationsachse gekrümmte Ausnehmungen, die wie Abflusskanäle wirken. Allerdings ist dadurch nicht sicher gewährleistet, dass dadurch auch der metallische Sägeblattabrieb vollständig aus dem Schneidenspalt nach außen geführt wird und so nicht in den Körper gelangt, was zu gesundheitlichen Komplikationen oder einer verzögerten Wundheilung führen kann.

Der Erfindung liegt deshalb die Aufgabe zugrunde, chirurgische Bearbeitungswerkzeuge für die Knochenchirurgie zu schaffen, bei denen ein dauerhafter Verbleib von metallischen Abriebrückständen oder Schneidenkanten im Körper sicher verhindert wird.

Diese Aufgabe wird durch die im Patentanspruch 1 angegebene Erfindung gelöst. Weiterbildungen und vorteilhafte Ausführungsbeispiele der Erfindung sind in den Unteransprüchen angegeben.

Die Erfindung hat den Vorteil, dass Rückstände von chirurgischen Bearbeitungswerkzeugen für Knochen aus einem biologischen Kunststoff auf Milchsäurebasis bestehen und deshalb nicht vollständig aus dem behandelten menschlichen Körper entfernt werden müssen, da diese vom Körper vollständig biologisch abgebaut werden. Dies ist insbesondere bei riotierenden oder oszillierenden Werkzeugen zur Knochenbearbeitung vorteilhaft, da bei diesen Rückstände in Form von Abriebpartikeln oder Schneidkantenresten nicht zu vermeiden sind, und aus dem menschlichen Körper dann nicht mehr entfernt werden können.

Dabei haben derartige Bearbeitungswerkzeuge aus einem biologisch abbaubaren Kunststoff aus Milchsäure zusätzlich den Vorteil, dass diese wie üblich thermoplastische Kunststoffe durch Spritzgießen, Thermoformen oder Extrusion in beliebigen Ausformungen einfach und kostengünstig herstellbar sind und so zu Sägeblättern, Bohrern oder Fräsern ausformbar sind. Da derartige Kunststoffe aus Milchsäure relativ hohe Glastemperaturen von mindestens 80° C aufweisen, können diese chirurgischen Werkzeuge auch mit spanabhebenden Verfahren bearbeitet werden, in dem besonders scharfe Bohr-, Fräs- oder Sägeschneiden herstellbar sind, die zur Knochenbearbeitung geeignet sind. Auch wenn diese Schneiden nicht so hohe Standzeiten aufweisen wie Metallschneiden, reicht dies für die einmalige Knochenbearbeitung aus, zumal diese Bearbeitungswerkzeuge kostengünstig herstellbar sind und daher in der Regel zum nur einmaligen Gebrauch vorgesehen sind. Da derartige biologische Kunststoffe auf Milchsäurebasis einen Schmelzpunkt von 150° bis 200°C besitzen, werden diese mit einer keimtötenden Temperatur hergestellt und sind deshalb ohne vorherige aufwendige Sterilisationsverfahren zum einmaligen Gebrauch einsetzbar.

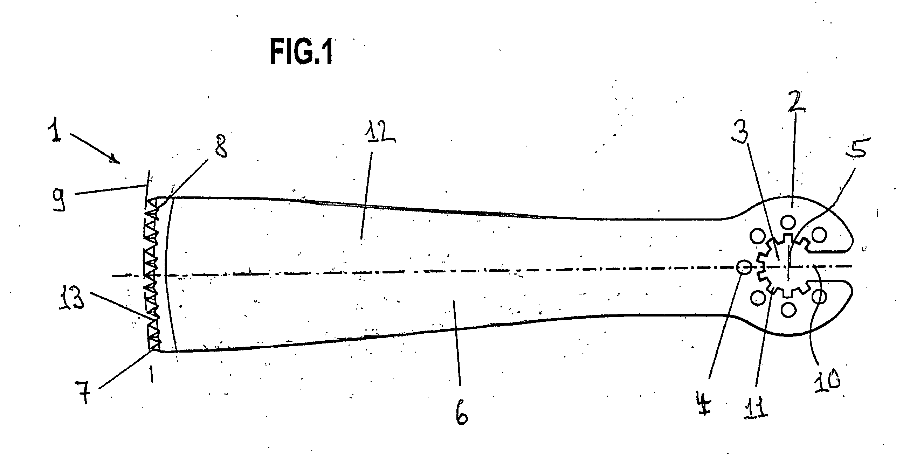

Die Erfindung wird anhand eines Ausführungsbeispiels, dass in der Zeichnung dargestellt ist, näher erläutert. Es zeigt die einzige
- Fig. 1:: ein chirurgisches Sägeblatt zur Knochenchirurgie.

In Fig. 1 der Zeichnung ist ein chirurgisches Sägeblatt 1 zur Knochenchirurgie dargestellt, das aus einem flachen langgestreckten Hauptkörper 6 aus einem biologisch abbaubaren Kunststoff als harten Werkstoff besteht. Dabei weist das Sägeblatt 1 am Ende einer Längsseite ein Halteelement 2 auf, das eine gezahnte runde Aussparung 3 mit Aussparungslücken 11 enthält, die in einen Antrieb mit einem Zahnstangenstumpf der Fa. Aesculap AG aus Tuttlingen einsetzbar ist. Koaxial zu dieser Aussparung 3 sind sieben Befestigungsbohrungen 4 angeordnet, die einen gleichgroßen Abstand zum Zentrum 5 der Aussparung 3 aufweisen. Da das Sägeblatt 1 vorzugsweise durch einen elektrischen Oszilliermotor angetrieben wird, stellt das Zentrum 5 der Aussparung 3 gleichzeitig eine Oszillationsachse dar, um die das Sägeblatt 1 mit einem Oszillationswinkel von ca. 1° bis 10° oszilliert. Das Halteelement 2 kann auch anders strukturierte Aussparungen 3 enthalten, die an Antriebe anderer Hersteller angepasst sind und z. B. als vierkant, achtkant, u-förmig oder in davon abgewandelter Form ausgebildet sind.

Am anderen Ende des Hauptkörpers 6 ist eine Sägezahnreihe 7 angeordnet, die das Bearbeitungselement zur Sägebearbeitung der Knochen darstellt. Dabei besteht die Sägezahnreihe 7 aus vorzugsweise dreizehn verschränkten Zähnen 8, deren äußere Begrenzungskante 9 eine Rundung um die Oszillationsachse 5 beschreibt. Das Sägeblatt 1 als oszillierendes Bearbeitungswerkzeug ist dabei insgesamt symmetrisch zu einer Längsachse 10 ausgebildet und vorzugsweise nur 0,5mm bis 3mm hoch. Dabei sind die einzelnen Zähne 8 dreieckförmig ausgebildet und nach außen spitz zulaufend ausgerichtet, Die Außenkanten 13 sind dabei als geneigte Schnittkanten ausgebildet.

Zur Knochenbearbeitung wird das Sägeblatt 1 mit seinem Halteteil 2 vorzugsweise auf einem akkubetriebenen oszillierenden Sägeblattantrieb befestigt. Dabei wird zum Zersägen des zu bearbeitenden Knochens das Sägeblatt 1 mit seiner Sägezahnreihe 7 auf den vorgesehenen Knochenteil aufgesetzt und der Oszillierantrieb eingeschaltet. Ein derartiger Sägevorgang durch das harte Knochenmaterial erzeugt dabei zwangsläufig einen Abrieb am Bearbeitungsteil 7, der bei herkömmlichen metallischen Sägeblattmaterialien nicht in die Wunde gelangen soll, so dass das erfindungsgemäße Sägeblatt 1 aus einem biologisch abbaubaren Werkstoff gefertigt ist, der vom menschlichen Körper ohne gesundheitliche Beeinträchtigungen abgebaut wird. Da dieser zur Knochenbearbeitung härter sein muss, wie der zu bearbeitende Knochen, wird dazu ein Werkstoff mit Milchsäure verwendet, der in einer derartigen Härte herstellbar ist und vom menschlichen Körper biologisch abgebaut wird. Hierbei handelt es sich um einen biologisch abbaubaren Kunststoff, der in fester Form vorliegt, die in Form von Polylactiden (PLA) bzw. Polymilchsäuren mit der chemischen Summenformel C₃H₆O₃ hergestellt werden. Sie gehören zu den Polyestern und sind aus vielen chemisch aneinander gebundenen Milchsäuremolekülen aufgebaut. Aus dem PLA können durch Wärmezufuhr verformbare Kunststoffe als Thermoplaste hergestellt werden. Dabei hängen deren Eigenschaften vor allem von der Molekülmasse, dem Kristallisierungsgrad und gegebenenfalls von dem Anteil der Co-Polymere ab. Eine höhere Molekülmasse steigert dabei die Glasübergangs- sowie die Schmelztemperatur, die Zugfestigkeit und das Elastizitätsmodul. Dabei werden Polylactide vorzugsweise durch die ionische Polymerisation von Lactid, einem ringförmigen Zusammenschluss von 2 Milchsäuremolekülen, erzeugt. Die Ringöffnungspolymerisation findet dabei bei Temperaturen zwischen 140° und 180°C sowie der Einwirkung katalytischer Zinnverbindungen statt. So werden Kunststoffe mit einer hohen Molekularmasse und Festigkeit erzeugt, die als Roh-Biokunststoffe als sogenannte PLA-Blends von verschiedenen Herstellerfirmen erhältlich sind. Aus derartigen PLA-Blends oder PLA-Granulaten sind dann durch typische Kunststoffverarbeitungsverfahren wie Thermoformen oder Spritzgießverfahren die Sägeblätter 1 herstellbar. Nach dem Aushärten der in Spritzgießformen hergestellten Sägeblätter 1 können diese zusätzlich auch angeschliffen werden, um die Schärfe zu erhöhen, wobei wegen der verhältnismäßig geringen Glastemperatur von 65° bis 85°C nur thermisch gering belastbare oder gekühlte Schleifverfahren anwendbar sind.

Zur Verbesserung der Härte der Polylactide (PLA) können diese auch Faserverstärkt werden und so Verbundwerkstoffe bilden. Bei der Verwendung von Naturfasern sind diese auch biologisch abbaubar. Zur Verbesserung der Bruchfestigkeit kann auch ein biologisch kompatibles biologisch abbaubares Wachs und anorganische Füllstoffe zugegeben werden. Hierfür kommen Wachse mit niedrigem Molekulargewicht aus der Gruppe der aliphatischen Polyester und Polyaminosäuren von vorzugsweise neun Gewichtsprozent in Betracht. Bei dem biologisch kompatiblen biologisch abbaubaren Anorganischen Füllstoffen eignen sich feine Pulver aus Keramikwerkstoffen aus Calciumphosphat, Hydroxyaphatit, Calciumsulfat, Calciumflorid, Calciumoxyd und Calciumcarbonat von vorzugsweise mindestens 30 Gewichtsprozenten. Hierdurch wird gleichzeitig auch die Gleiteigenschaft verbessert, wodurch sich die Temperatur am Sägeblatt 1 verringern lässt.

Die Temperaturbeständigkeit kann aber auch dadurch erhöht werden, indem bei den biologisch abbaubaren Kunststoffen vorzugsweise aus rechtsdrehenden Polylactiden (L-Milchsäure) diese mit linksdrehender Milchsäure (D-Milchsäure) verbessert werden. Außerdem lässt sich durch eine gleichzeitige Verstärkung mit Naturfasern die Temperaturbeständigkeit in einen Bereich von etwa 100°C erhöhen.

Zur Verringerung der Temperaturbelastung kann gleichzeitig auch der Oszillationsantrieb auf eine relativ geringe Oszillationsfrequenz eingestellt werden, was gleichzeitig auch die Abnutzung des Sägeblattes 1 reduziert. Wegen der relativ geringen Härte dieser biologisch abbaubaren Kunststoffe aus Milchsäuren sind diese vorzugsweise nur zum einmaligen Gebrach ausgelegt, wodurch sich auch eine aufwendige Sterilisation vermeiden lässt.

## Patentansprüche

1. Chirurgisches Bearbeitungswerkzeug (1) für die Knochenchirurgie, dass ein Halteelement (2) zur Verbindung mit einem Antrieb und mindestens einem Bearbeitungselement (7) zur spanabhebenden Bearbeitung von Knochen enthält, wobei mindestens das Bearbeitungselement (7) aus einem harten Werkstoff besteht, **dadurch gekennzeichnet, dass** mindestens das Bearbeitungselement (7) aus einem biologisch abbaubaren Werkstoff besteht, der Milchsäure enthält.

2. Chirurgisches Bearbeitungswerkzeug für die Knochenchirurgie nach Anspruch 1, **dadurch gekennzeichnet, dass** der biologisch abbaubare Werkstoff als fester biologischer Kunststoff ausgebildet ist, der Polylactide (PLA) nach der chemischen Summenformel C₃H₆O₃ enthält.

3. Chirurgisches Bearbeitungswerkzeug für die Knochenchirurgie nach Anspruch 1 oder 2, **dadurch gekennzeichnet, dass** die Polylactiden Polymere darstellen, die aus rechtsdrehenden L-Lactiden oder aus rechtsdrehenden L-Lactiden mit zugesetzten linksdrehenden D-Lactiden bestehen.

4. Chirurgisches Bearbeitungswerkzeug für die Knochenchirurgie, nach einem der vorhergehenden Ansprüche, **dadurch gekennzeichnet, dass** der biologische Kunststoff (PLA) zusätzlich noch biologisch abbaubare Naturfasern enthält.

5. Chirurgisches Bearbeitungswerkzeug für die Knochenchirurgie, nach einem der vorhergehenden Ansprüche, **dadurch gekennzeichnet, dass** der biologische Kunststoff aus Polylactiden zusätzlich ein biologisch abbaubares Wachs und ein biologisch abbaubares Füllstoffmaterial enthält.

6. Chirurgisches Bearbeitungswerkzeug für die Knochenchirurgie, nach einem der vorhergehenden Ansprüche, **dadurch gekennzeichnet, dass** das Bearbeitungswerkzeug (1) als oszillierbares Sägeblatt (1) ausgebildet ist, dass einen axial langgestreckten Hauptkörper (6) enthält, an dessen einem Ende eine Sägezahnreihe (7) als Bearbeitungselement und an dessen gegenüberliegenden anderen Ende eine Aussparung (3) als Halteelement (2) angeordnet ist.

7. Chirurgisches Bearbeitungswerkzeug für die Knochenchirurgie, nach Anspruch 6, **dadurch gekennzeichnet, dass** das Sägeblatt (1) symmetrisch zu einer Längsachse (10) ausgebildet ist wobei die Sägezahnreihe (7) im Wesentlichen quer zur Längsachse (10) ausgerichtet ist und der Schnittpunkt der Längsachse (10) mit dem Zentrum (5) der Aussparung (3) eine Oszillationsachse für den Antrieb bildet.

8. Chirurgisches Bearbeitungswerkzeug für die Knochenchirurgie, nach einem der vorhergehenden Ansprüche, **dadurch gekennzeichnet, dass** der Hauptkörper (6), die Sägezahnreihe (7) und das Halteelement (2) aus einem flachen Längskörper mit parallelen planen Außenflächen (12) besteht.

9. Chirurgisches Bearbeitungswerkzeug für die Knochenchirurgie, nach einem der vorhergehenden Ansprüche, **dadurch gekennzeichnet, dass** die Sägezahnreihe (7) aus mindestens 10 nach unten spitz zulaufenden dreieckförmigen Zähnen (8) besteht, wobei die nach unten zulaufenden Außenkanten (13) als Schnittkanten ausgebildet sind.
